# EUROPEAN PATENT SPECIFICATION

(11) **EP 2 464 229 B1**
(45) Date of publication and mention of the grant of the patent: **25.11.2015**
(21) Application number: 10808713.1
(22) Date of filing: 11.08.2010
(51) Int. Cl.: A61K 31/4409, A61K 31/44, A61K 45/06, A61K 9/20, A61K 9/48, A01N 43/40, A61P 25/00

(54) **USE OF 4-AMINOPYRIDINE TO IMPROVE NEURO-COGNITIVE AND/OR NEURO-PSYCHIATRIC IMPAIRMENT IN PATIENTS WITH MULTIPLE SCLEROSIS**
VERWENDUNG VON 4-AMINOPYRIDIN ZUR LINDERUNG NEUROKOGNITIVER UND/ODER NEUROPSYCHIATRISCHER BEEINTRÄCHTIGUNGEN BEI PATIENTEN MIT MULTIPLER SKLEROSE
UTILISATION DE LA 4-AMINOPYRIDINE POUR AMÉLIORER UN DYSFONCTIONNEMENT NEUROCOGNITIF ET/OU NEUROPSYCHIATRIQUE CHEZ DES PATIENTS ATTEINTS DE SCLÉROSE EN PLAQUES

(30) Priority: 11.08.2009 US 233077 P; 11.08.2009 US 233069 P; 04.09.2009 US 239877 P
(43) Date of publication of application: 20.06.2012
(73) Proprietor: Acorda Therapeutics, Inc., Ardsley, NY 10502 (US)
(72) Inventor: WESSEL, Thomas, C., Lenox MA 01240 (US); BLIGHT, Andrew, Mahopac NY 10541 (US)
(74) Representative: Goodfellow, Hugh Robin
(86) International application number: PCT/US2010/045211
(87) International publication number: WO 2011/019845

(56) References cited:
- WO-A2-02/072025
- US-A1- 2005 228 030
- US-A1- 2005 276 851
- KORENKE ANNE R ET AL: "Sustained-release fampridine for symptomatic treatment of multiple sclerosis", THE ANNALS OF PHARMACOTHERAPY,, vol. 42, no. 10, 1 October 2008 (2008-10-01), pages 1458-1465, XP009163811, ISSN: 1542-6270
- POLMAN C H: "4 - AMINOPYRIDINE IN THE TREATMENT OF PATIENTS WITH MULTIPLE - SCLEROSIS - LONG - TERM EFFICACY AND SAFETY", ARCHIVES OF NEUROLOGY, AMERICAN MEDICAL ASSOCIATION, CHICAGO, IL, US, vol. 51, no. 3, 1 March 1994 (1994-03-01), pages 292-296, XP009165286, ISSN: 0003-9942
- Solari, A. et al.: "Aminopyridines for symptomatic treatment in multiplesclerosis (Review)", The Cochrane Collaboration , 21 January 2009 (2009-01-21), XP002689390, Retrieved from the Internet: URL:http://onlinelibrary.wiley.com/doi/10. 1002/14651858.CD001330/pdf/standard [retrieved on 2012-12-17]
- Wallin, M. et al.: "Cognitive dysfunction in multiple sclerosis: Assessment, imaging,and risk factors", VA Research and Development Journal of Rehabilitation Research and Development, vol. 43, no. 1 January 2006 (2006-01), pages 63-72, XP002689391, Retrieved from the Internet: URL:http://www.rehab.research.va.gov/jour/ 06/43/1/page63.html [retrieved on 2012-12-17]
- Lovera, J. et al.: "Correlations of Perceived Deficits Questionnaire of Multiple Sclerosis Quality of Life Inventory with Beck Depression Inventory and neuropsychological tests", VA Research and Development Journal of Rehabilitation Research and Development, vol. 43, no. 1 January 2006 (2006-01), pages 73-82, XP002689392, Retrieved from the Internet: URL:http://www.rehab.research.va.gov/jour/ 06/43/1/Lovera.html#top [retrieved on 2012-12-17]
- BEGLINGER L J ET AL: "Practice effects and the use of alternate forms in serial neuropsychological testing", ARCHIVES OF CLINICAL NEUROPSYCHOLOGY, PERGAMON, vol. 20, no. 4, 1 June 2005 (2005-06-01), pages 517-529, XP027831954, ISSN: 0887-6177 [retrieved on 2005-06-01]
- PEDRAZA O ET AL: "P1-135 Factor validity and cross-cultural equivalence of a core neuropsychological test battery in elderly African Americans", NEUROBIOLOGY OF AGING, TARRYTOWN, NY, US, vol. 25, 1 July 2004 (2004-07-01), page S134, XP027081936, ISSN: 0197-4580 [retrieved on 2004-07-01]

## Description

The present application claims benefit of priority to U.S. Provisional Application Serial No. 61/233,069 filed August 11, 2009; U.S. Provisional Application No. 61/233,077 filed August 11, 2009; and U.S. Provisional Application Serial No. 61/239,877 filed September 4, 2009.

### FIELD OF THE INVENTION

The present invention relates to conditions that affect brain function. In a particular embodiment, the invention relates to use of sustained release 4-aminopyridine to improve or stabilize the neuro-cognitive and/or neuro-psychiatric impairment of patients with Multiple Sclerosis (MS).

### BACKGROUND OF THE INVENTION

MS is thought to be an autoimmune disease and is characterized by areas of demyelination (lesions) in the CNS. This characteristic demyelination and associated inflammatory response lead to abnormal impulse conduction or conduction block in nerve fibers traversing the lesions. Lesions can occur throughout the CNS but certain sites such as the optic nerve, brainstem, spinal cord, and periventricular region seem particularly vulnerable. Impaired action potential conduction is probably the major contributor to the symptoms most often reported (e.g., paralysis, visual abnormalities, muscle weakness, nystagmus, sensory abnormalities, and speech disturbances).

With advances in the understanding of medical conditions, cognitive dysfunction has come to be seen as a significant issue for patients with MS. Controlled neuropsychological studies have shown that a substantial proportion of multiple sclerosis patients experience cognitive dysfunction. Recent memory, sustained attention, conceptual-abstract reasoning, and speed of information processing are impaired in about 50% of patients, whereas language functions are relatively spared. (Rao SM, Reingold SC, Ron MA, Lyon-Caen 0, Comi G. Conference report: workshop on neurobehavioral disorders in multiple sclerosis. Arch Neurol 1993;50;658-662.) Rao et al demonstrated that a subgroup of MS patients with cognitive dysfunction were less likely to be employed, less likely to engage in social and recreational activities, and required greater personal assistance than a subgroup of cognitively intact patients with MS, despite both subgroups having an equivalent degree of physical disability. (Rao SM, Leo GJ, Ellington L, Nauertz T, Bernardin L, Unverzagt F. Cognitive dysfunction in multiple sclerosis. 11. Impact on employment and social functioning. Neurology 1991;41:692-696.)

Studies of fampridine (4-aminopyridine) have been conducted using intravenous (i.v.) administration and immediate-release (IR) oral capsule formulations in addition to controlled-release or sustained-release formulations. Administration of IR capsules resulted in rapid and short-lasting peaks of fampridine in the plasma. Early pharmacokinetic studies were conducted using an immediate release (IR) formulation for oral administration, which consisted of fampridine powder in a gelatin-based capsule or oral solution. Administration resulted in rapidly changing fampridine plasma levels that were not well tolerated. A sustained-release matrix tablet (Fampridine-SR) was then developed. The Fampridine-SR matrix tablet showed improved stability and an appropriate pharmacokinetic profile for twice-daily dosing.

Studies in people with multiple sclerosis (MS), including Phase 1, 2 and 3 clinical trials, indicate that the drug fampridine improves a variety of neurological functions that are impaired by this disease. US 2005/0228030 describes a sustained release composition of 4-aminopyridine for use in treating various neurological diseases, including multiple sclerosis. Polman et al (Polman CH, et al. 4-aminopyridine in the treatment of patients with multiple sclerosis. Arch Neurol 1994;51;292-296.) describes a non-sustained release 4-aminopyridine for use in the treatment of thirty-one patients with multiple sclerosis. Solari et al (Solari A, et al. Aminopyridines for symptomatic treatment in multiple sclerosis (Review). The Cochrane collaboration, 21 January 2009) reviews clinical trials to determine the efficacy and safety of aminopyridines for neurological deficits in adults with MS.

Current terminology characterizes symptomatic MS by either a relapsing course or a more severe progressive course. The following Table 1 presents the four clinical subtypes and their prevalence/incidence. Note that incidence for the first three subtypes reflects disease onset. The clinical course of secondary-progressive MS is always preceded by relapsing-remitting disease.

**Table 1: Clinical Subtypes of MS**

| **MS Subtype** | **Prevalence/Incidence** |
|---|---|
| Relapsing-remitting (RR) MS | 85% |
| Primary-progressive (PP) MS | 10% |
| Progressive-relapsing (PR) MS | 5% |
| Secondary-progressive (SP) MS | 30% of all MS patients; up to 80% of untreated relapsing MS |

There remains a need in the art for methods of ameliorating the problem of brain effects such as cognitive impairment in MS, as well as in other patient populations subject to demyelinating and traumatic conditions.

### SUMMARY OF THE INVENTION

In one aspect, the invention relates to a sustained release composition of 4-aminopyridine for use in a method of dosing to improve at least one neuro-cognitive or neuro-psychiatric parameter in a patient, wherein the patient has MS. Also disclosed is use of an aminopyridine, such as fampridine or Fampridine-SR, in a dosing regimen that serves to improve or stabilize a mental status parameter in a patient. The patient has MS. In particular, a dosing regimen is disclosed that is found to elicit improvement(s) in one or more brain function parameter, such as depression, libido, euphoria, a decrease in mentation, fatigue or cognitive impairment. In certain embodiments, the composition for use according to the invention are for use, prescription, administration in combination with another therapeutic modality to address a neuro-cognitive or neuro-psychiatric disorder; another therapeutic modality to address a neuro-cognitive or neuro-psychiatric disorder can comprise other drugs, regimens, protocols and/or psychological or psychiatric treatments known to those of skill in the art. In one embodiment of a composition for use in a method of the invention, the method comprises administering wherein the patient has MS; the method comprising administering in combination with another therapeutic modality to address MS.

The composition for use in the methods set forth herein is useful in each of the 4 clinical subtypes of MS. The composition for use in the methods set forth herein is useful in Relapsing-remitting (RR) MS. The composition for use in the methods set forth herein is useful in Primary-progressive (PP) MS. The composition for use in the methods set forth herein is useful in Progressive-relapsing (PR) MS. The composition for use in the methods set forth herein is useful in Secondary-progressive (SP) MS. The composition for use in the methods set forth herein is useful in progressive and non-progressive disease, the composition for use in the methods herein is useful in temperature sensitive patients and patients whom do not have temperature sensitive disease. The composition for use in the methods set forth herein is effective without distinction as to the duration of MS illness. The composition for use in the methods set forth herein is effective without distinction as to the severity of MS. The composition for use in the methods set forth herein is useful when other symptoms of MS are not affected in a clinically meaningful way by any aminopyridine treatment.

In the description, figures and tables herein, a number of terms are used. In order to provide a clear and consistent understanding of the specification and claims, the following definitions are provided:

### DEFINITIONS:

As used herein, the term "about" means plus or minus 15, 14, 13, 12, 11, 10% or less than 10% of the value with which it is being used. "About" is inclusive. Therefore, in one example where about means 10%, "about 50%" means in the range of 45%-55% inclusive. It is within the scope of the present invention that a value "about" that of any of the ng/ml values set forth herein is within the scope of the invention; it is to be understood that, without limitation a value "about" a particular ng/ml includes plus or minus 0.6, 0.5. 0.4, 0.3, 0.2 or 0.1 ng/ml.

When used in conjunction with the word "comprising" or other open language in the claims, the words "a" and "an" denote "one or more."

"Administering" when used in conjunction with a therapeutic means to administer a therapeutic directly into or onto a target tissue or to administer a therapeutic to a patient whereby the therapeutic positively affects or impacts or influences the tissue to which it is targeted. Thus, as used herein, the term "administering", when used in conjunction with a compound, can comprise but is not limited to, providing a compound into or onto the target tissue; providing a compound systemically to a patient by, e.g., intravenous injection (e.g., parenteral) or oral administration (e.g., enteral) or topical (e.g., transdermal, transcutaneous, patch, suppository) or inhalation (e.g., transmucosal) administration, whereby the therapeutic reaches the target tissue. "Administering" a composition may be accomplished by various techniques as described herein. Further "administering" refers to the act of giving or providing a composition or compound to a patient by the patient himself or herself or by a caregiver, such as a medical professional; including the act of ingestion by or application to the patient or the like wherein the composition or compound can exert its effects.

The term "animal" as used herein includes, but is not limited to, humans and non-human vertebrates such as wild, domestic and farm animals.

In addition, the "compounds" of the present invention can exist in unsolvated as well as solvated forms with pharmaceutically acceptable solvents such as water, ethanol, and the like. In general, the solvated forms are considered equivalent to the unsolvated forms for the purposes of the present invention.

The term "improvement" designates an alteration in a parameter in a desired direction. As used herein, "improvement" also comprises stabilization of a parameter that would otherwise be deteriorating or moving in a non-desired direction.

The term "inhibiting" includes the administration of a compound of the present invention to prevent the onset of the symptoms, alleviating the symptoms, or eliminating the disease, condition or disorder.

"Local administration" means direct administration by a non-systemic route at or in the vicinity of the site of affliction, disorder, or perceived pain.

By "pharmaceutically acceptable", it is meant the carrier, diluent or excipient must be compatible with the other ingredients of the formulation and not deleterious to the recipient thereof.

The term "prodrug" refers to compounds that are rapidly transformed in vivo to yield the parent compounds of the above formula, for example, by hydrolysis in blood. A thorough discussion is provided in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

The terms "patient" and "subject" mean animals including mammals, and in one embodiment humans. Examples of patients or subjects include humans, cows, dogs, cats, goats, sheep, and pigs.

As used herein, the term "Responder" is generally a statistical term, and is not intended to reflect the existence or lack thereof of utility or enablement for an outcome of the invention. Accordingly, an individual can obtain a useful response to a method of the invention but not at the same time meet a particular set of statistical criteria as a "Responder."

The term "salts" refers to the relatively non-toxic, inorganic and organic acid addition salts of compounds of the present invention. These salts can be prepared in situ during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate mesylate, glucoheptonate, lactobionate and laurylsulphonate salts, and the like. These may include cations based on the alkali and alkaline earth metals, such as sodium, lithium, potassium, calcium, magnesium, and the like, as well as non-toxic ammonium, tetramethylammonium, tetramethylammonium, methlyamine, dimethlyamine, trimethlyamine, triethlyamine, ethylamine, and the like. (See, for example, S.M. Barge et al., "Pharmaceutical Salts," J. Pharm. Sci., 1977, 66:1-19).

As used herein, the term "steady state" indicates a system that has one or more properties that are unchanging over time or "steady state" indicates a system that has one or more properties that are changing within a limited range over time. Typically, steady state is a more general situation than dynamic equilibrium. If a system is in steady state, then the recently observed behavior of the system will generally continue into the future. In many systems, steady state is not achieved until some time has elapsed after the system is started or initiated. This initial situation is often identified as a transient state, titration period, start-up or warm-up period.

As used herein, the term "sustained-release" as it relates to the aminopyridine compositions includes the release of a aminopyridine from the dosage formulation at a sustained rate such that a therapeutically beneficial blood level maintained over a period of at least about 6, 7, 8, 9, 10, 11, 12, 13, 14, 15, 16, 17, 18, 19, 20, 21, 22, 23, 24, 25, 26, 27, 28, 29, 30, hours, or more than 18 hours, or more than 24 hours, or more than 30 hours. Preferably, the amount of the aminopyridine in the oral dosage formulations according to embodiments of the present invention establish a therapeutically useful plasma or CNS concentration through t.i.d., b.i.d., or q.d. administration of the pharmaceutical composition. The terms "sustained release" and "extended release" are generally synonymous unless the context clearly indicates otherwise.

As used herein, the term "therapeutic" means an agent utilized to treat, combat, ameliorate, palliate, prevent or improve an unwanted condition or disease of a patient. In part, embodiments of the present invention are directed to the treatment of multiple sclerosis and/or any symptom thereof. In part, embodiments of the present invention are directed to the process of achieving a therapeutic outcome in multiple sclerosis and/or any symptom thereof.

A "therapeutically effective amount" is an amount sufficient to achieve a treatment or a therapeutic outcome. In one embodiment, a "therapeutically effective" amount of compound optionally includes a physiologically tolerable excipient(s). In one embodiment, a "therapeutically effective" amount is sufficient to achieve an effective systemic concentration or local concentration in the tissue. In one embodiment, a "therapeutically effective" amount is sufficient to achieve improvement in one or more symptoms known to be associated with the disease multiple sclerosis; such symptoms include without limitation: autonomic functions, bladder dysfunction, bowel dysfunction, sexual dysfunction, strength, energy, pain, weakness and fatigue (endurance impairment), muscle weakness, sensory and motor symptoms, paresthesia, tremor, speech deficits, altered range of motion, vision, visual disturbances and eye movement disorders, coordination and balance symptoms, ataxia, fine hand coordination, upper extremity function, walking, spasticity, cognitive and mental disorders, mood, cognition, and/or psychiatric/psychological factors.

As used herein, "treatment" comprises any of: an ongoing process or outcome that ameliorates, palliates, decreases or prevents the symptoms associated with a medical condition or infirmity; an ongoing process or outcome that improves the symptoms associated with a medical condition or infirmity; an ongoing process or outcome to normalize body functions in disease or disorders that result in impairment of specific bodily functions; or an ongoing process or outcome that elicits an improvement in one or more of the clinically measured parameters of the disease. In one embodiment, a treatment objective is to prevent or slow down (lessen) an undesired physiological condition, disorder or disease, or to obtain beneficial or desired result. The result can be, e.g., medical, physiological, clinical, physical therapy, occupational therapy, subjective to a health care worker or to a patient; or a parameter understood in the art as a "quality of life" or an "activity of daily living". For the purposes of this invention, beneficial or desired clinical results comprise, but are not limited to, alleviation of symptoms; diminution/diminishment of the extent of the condition, disorder or disease; stabilization (*i.e.,* not worsening) of the state of the condition, disorder or disease; delay in onset or slowing of the progression of the condition, disorder or disease; amelioration or palliation of the condition, disorder or disease; and remission (whether partial or total), whether detectable or undetectable; or enhancement or improvement of the condition, disorder or disease. In one embodiment, treatment includes eliciting a clinically significant response without excessive levels of side effects. In one embodiment, treatment also includes prolonging survival as compared to expected survival if not receiving treatment. In one embodiment, treatment refers to the administration of medicine or the performance of medical procedures with respect to a patient. As used herein, treatment can be to prophylax (prevention), to cure the infirmity or malady, or to ameliorate the clinical condition of the patient, including a decreased duration of illness or severity of illness, or subjective improvement in the quality of life of the patient or a prolonged survival of the patient.

Moreover, the terms "treat," "treated," "treatment", "treating" or "therapy" generally are synonyms unless the context indicates otherwise; as used herein these broadly refer to any of therapeutic, prophylactic or preventative, or curative measures. It is to be understood that one or more embodiments of "treat," "treated," "treatment", "treating", "therapeutic" or "therapeutically effective" can occur together.

Generally speaking, the term "tissue" refers to any aggregation of similarly specialized cells that are united in the performance of a particular function.

| **Abbreviation or Specialist Term** | **Explanation** |
|---|---|
| ADME | Absorption, distribution, metabolism, and excretion |
| Aₑ | Amount of drug excreted |
| APD₃₀, APD₅₀, APD₉₀ | Action potential duration 30%, 50%, 90% |
| AUC | Area under the concentration-time curve |
| AUC₍₀₋ₜ₎, AUC_{(0-∞)} or AUC_{(0-inf)} | Area under the plasma concentration *versus* time curve, to the last quantifiable level, and extrapolated to infinity |
| AUC₍₀₋₁₂₎, AUC₍₀₋₂₄₎ | Area under the plasma concentration *versus* time curve, 0-12 hours, 0-24 hours |
| b.i.d. (bid) | Twice daily |
| ¹⁴C | Radioactive carbon 14 |
| CHO | Chinese hamster ovary |
| CI | Confidence interval |
| CL/F | Apparent total body clearance after administration |
| Cl_{R} | Renal clearance |
| Cm | Centimeter |
| Cₘₐₓ | Maximum measured plasma concentration |
| CNS | Central nervous system |
| CR | Controlled-release |
| CrCl | Creatinine clearance |
| CumAₑ | Cumulative amount of drug excreted |
| CYP, CYP 450 | Cytochrome p450 isoenzymes |
| ECG | Electrocardiogram |
| EEG | Electroencephalogram |
| F | Female |
| FOB | Functional Observation Battery |
| 4-AP | 4-Aminopyridine (also known as fampridine or dalfampridine) |
| g, kg, mg, µg, ng | Gram, kilogram, milligram, microgram, nanogram |
| GABA | Gamma-aminobutyric acid |
| GLP | Good Laboratory Practice |
| h, hr | Hour |
| HDPE | High-density polyethylene |
| hERG | Human ether-à-go-go related gene |
| HPLC | High performance liquid chromatography |
| IC₅₀ | 50% Inhibitory concentration |
| I_{Kr} | Potassium ion channel whose activity is measured in the hERG assay |
| Improvement | Designates an alteration in a parameter in a desired direction. As used herein, "improvement" also comprises stabilization of a parameter that would otherwise be deteriorating or moving in a non-desired direction. |
| IND | Investigational New Drug application |
| IR | Immediate-release |
| i.v. (iv) | Intravenous |
| K⁺ | Potassium |
| Kₑₗ | Elimination constant |
| L, mL | Liter, milliliter |
| LCMS, LC/MS/MS | Liquid chromatography / mass spectrometry |
| LD₅₀ | Median lethal dose |
| Ln | Natural log |
| LOQ | Limit of quantitation |
| M | Male |
| Min | Minute |
| mM, µM | Millimolar, micromolar |
| MRT | Mean residence time |
| MS | Multiple sclerosis |
| MTD | Maximum tolerated dose |
| NA | Not applicable |
| ND | None detected |
| NDA | New Drug Application |
| NE | Not evaluable |
| NF | National Formulary |
| NOAEL | No observable adverse effect level |
| NOEL | No observable effect level |
| Norm | Normalized |
| NZ | New Zealand |
| pₐₚₚ | Apparent permeability coefficient |
| p.o. | Oral |
| SAE | Serious adverse event |
| SCI | Spinal cord injury |
| SD | Standard deviation |
| Sec | Second |
| SEM | Standard error of the mean |
| SPF | Specific pathogen-free |
| SR | Sustained-release |
| SS | Steady state |
| t_{1/2} | Apparent terminal elimination half-life |
| t.i.d. (tid) | Three times daily |
| TK | Toxicokinetics |
| TLC | Thin layer chromatography |
| Tₘₐₓ | Time of the maximum measured plasma concentration |
| USP | United States Pharmacopeia |
| UTI | Urinary tract infection |
| V_{d} | Volume of distribution |
| V_{dss} | Volume of distribution at steady state |

### BRIEF DESCRIPTION OF THE DRAWINGS

The following drawings form part of the present specification and are included to demonstrate certain aspects of the present disclosure in greater detail. The invention may be better understood by reference to one of these drawings in combination with the detailed description of specific embodiments presented herein.

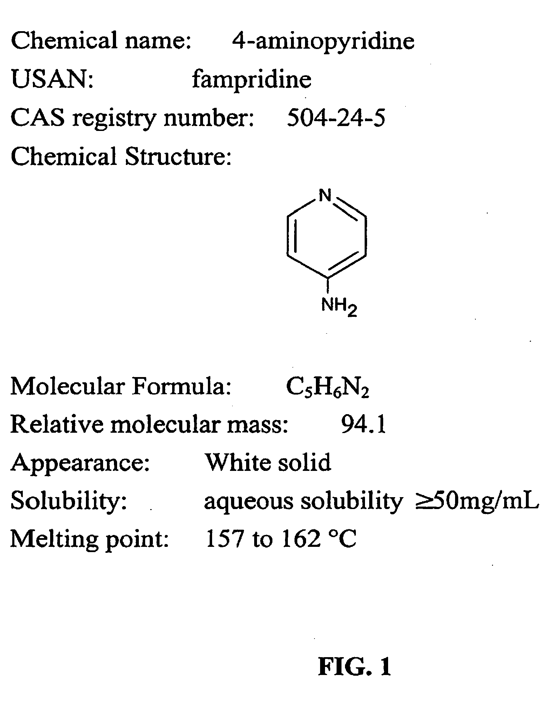

FIG. 1 shows information regarding fampridine.
FIG. 2 shows information regarding exemplary test components of Cognitive Assessment Protocol for MS.
FIG 3. Data for ten patients. For these patients, mean improvement = -0.183 ± 0.137; P = 0.05 (2-tailed t-test). EDSS = Expanded Disability Status Scale; RR = relapsing-remitting; SP = secondary progressive.

### DETAILED DESCRIPTION OF THE INVENTION

Fampridine is the common name for the coumpound 4-aminopyridine (4 AP). Fampridine is a potassium (K+) channel blocker that has been successfully identified as a treatment for improving neurological and muscular function in patients with Multiple Sclerosis (MS). Fampridine is known as dalfampridine in the U.S. because dalfampridine is the United States Adopted Name (USAN) for the chemical 4-aminopyridine (4 AP). Fampridine has a molecular formula of C₅H₆N₂ and molecular weight of 94.1. Throughout this specification the terms "fampridine", "dalfampridine" and "4-aminopyridine" are and can be used interchangeably to refer to the active drug substance. Fampridine has been formulated as a sustained-release (SR) matrix tablet in various strengths from 5 to 40 mg.

Fampridine-SR (available in the U.S., under the tradename Ampyra®, Acorda Therapeutics, Hawthorne NY), is available in a strength of 10 mg tablets. In one embodiment, the following excipients are generally included in each tablet: hydroxypropyl methylcellulose, USP; microcrystalline cellulose, USP; colloidal silicon dioxide, NF; magnesium stearate, USP; and Opadry White.

Pharmacologically, the K+ channel blocking properties of 4-aminopyridine and its effects on action potential conduction in demyelinated nerve fiber preparations have been extensively characterized. At low concentrations that are relevant to clinical experience, in the range of 0.2 to 2 µM (18 to 180 ng/mL), 4-aminopyridine is able to block certain voltage-dependent K+ channels in neurons. It is this characteristic that appears to explain the ability of the drug to restore conduction of action potentials in demyelinated nerve fibers. At higher (millimolar) concentrations, fampridine affects other types of K+ channels in both neural and non-neural tissues. Blockade of repolarizing K+ currents can increase synaptic transmission throughout the nervous system by increasing the duration of the pre-synaptic action potential. A range of neurological effects consistent with increased excitability of presynaptic nerve terminals occurs with clinically relevant doses of fampridine.

### Effects on Axonal Conduction Block

The K+ channels blocked by low concentrations of 4-aminopyridine are partially responsible for repolarization of neuronal action potentials. These appear to include those found under the myelin sheath in myelinated nerve fibers of adult mammals. These channels are located primarily in the paranodal and internodal membrane of the axon (Waxman and Ritchie, 1993) where they are not significantly activated by the passage of an action potential because the myelin sheath acts as an electrical shield. Therefore, the action potential of normal adult myelinated axons shows little or no sensitivity to 4-aminopyridine at concentrations below 100 µM (9.4 µg/mL) (Shi and Blight, 1997). Concentrations above 1 mM (94.1 µg/mL) tend to cause gradual depolarization of the axon resting potential, perhaps by interacting with leakage channels (Shi and Blight, 1997).

When the axon is demyelinated, the internodal membrane and its ion channels become exposed to larger electrical transients during the action potential. Leakage of ionic current through the K+ channel, under these conditions, can contribute to the phenomenon of action potential conduction block (Waxman and Ritchie, 1993). 4-Aminopyridine may prolong nerve action potentials by blocking these exposed channels and inhibiting repolarization (Sherratt et al., 1980). This is consistent with the ability of the drug to overcome conduction block and increase the safety factor for conduction in some critically demyelinated axons (Bostock et al., 1981; Targ and Kocsis, 1985) including those in chronically injured and partially remyelinated mammalian spinal cord (Blight, 1989; Shi and Blight, 1997). An additional study (Shi et al., 1997) showed that this effect of 4-aminopyridine in the chronically injured spinal cord of guinea pigs occurs at a concentration threshold between 0.2 to 1 µM (19.1 to 94.1 ng/mL), though in this tissue it is most effective at about 10 µM (941 ng/mL).

Repetitive impulse activity, either spontaneous or in response to single stimuli, occurs in some demyelinated axons exposed to higher levels [0.1 to 1 mM (9.4 to 94.1 µg/mL)] of 4-aminopyridine in vitro (Blight, 1989; Bowe et al., 1987; Targ and Kocsis, 1985). A similar effect at lower concentrations on susceptible neurons or nerve endings may explain the paresthesias and pain in the area of intravenous infusion that have been reported as side effects of clinical exposure to 4-aminopyridine in human subjects. However, there are no published data to indicate that repetitive spontaneous activity occurs in such nerve fibers with lower, clinically relevant concentrations in the range of 0.25 to 1 µM (23.5 to 94.1 ng/mL).

It is understood that blockade of K+ currents amplifies synaptic transmission throughout the brain and spinal cord. A range of neurological effects occurs with increasing concentrations of 4-aminopyridine in the central nervous system (CNS), up to and including the initiation of seizures. Various in vitro brain slice experiments have shown epileptiform discharges in the amygdala (Gean, 1990) and hippocampus (Rutecki et al., 1987) of rats when the tissue was superfused with solutions containing 5 to 500 µM (0.47 to 47 µg/mL) 4-aminopyridine. Seizure activity in animals has been seen following large doses of 4-aminopyridine, and seizure activity is part of the toxicological profile of the drug. Synchronous bursting activity in the spinal cord of decerebrate cats has been recorded following administration of very large doses of 4-aminopyridine (5 to 20 mg/kg), which would be expected to produce plasma levels in the region of several hundred ng/mL (Dubuc et al., 1986). For the first time herein, these neurological effects are disclosed to be an aspect in the treatment of neuro-cognitive impairment (and related neuro-psychiatric issues), and are overcome by methods in accordance with the invention.

### Absorption

4-Aminopyridine is rapidly absorbed following oral administration. In an in situ study, 4-aminopyridine was more rapidly absorbed from the small intestine than from the stomach. The absorption half-life was 108.8 minutes and 40.2 minutes for the stomach and small intestine, respectively. In an in vitro study with vascularly perfused rat gut segments, the regional apparent permeability coefficient (pₐₚₚ × 10⁻⁶, cm/sec) of 4-aminopyridine was high in the upper small intestine (22.7 cm/sec) and decreased distally towards the large intestine (2.9 cm/sec) compared to a poorly permeable marker (atenolol; 1.9 cm/sec in the upper small intestine and 0 cm/sec in the large intestine) (Raoof et al., 1997).

Following oral administration of (non-sustained release) 4-aminopyridine in animals, peak plasma concentrations occur within 1 hour of dosing. Based on comparisons of the areas under the plasma concentration-versus-time curve (AUC_{(0-∞)}) following i.v. and p.o. administration of 4-aminopyridine (2 mg/kg), the bioavailability of 4-aminopyridine was reported to be approximately 66.5% in male rats and 55% in female rats (M 2001-03). Following oral administration, peak plasma concentrations were 38% lower in females than in males, although both (AUC_{(0-∞)}) and body weight were similar; AUC values did not differ between males and females following i.v. administration.

Studies were performed in rats and dogs using ¹⁴C-labeled 4-aminopyridine (1 mg/kg) given as a single oral gavage dose in solution. In both species, 14C 4-aminopyridine was rapidly absorbed. Peak plasma levels were achieved within 0.5 to 1 hour in both species. The peak plasma levels (Cmax) and the extent of absorption as reflected by the AUC were both approximately four-fold higher in the dog than in the rat following doses equal on a mg/kg basis. In these studies, there were no gender differences evident in either species. These results are summarized in Table 1.

**Table 1: Summary of Absorption Data for Rats and Dogs Following Single Oral Administration of ¹⁴C-4-Aminopyridine 1 mg/kg (Study Nos. HWI 6379-101 and HWI 6379-102)**

| **Parameter** | **Rats (Study HWI 6379-101)** | | **Dogs (Study HWI 6379-102)** | |
|---|---|---|---|---|
| | **Males (N=3¹)** | **Females (N=3¹)** | **Males (N=3)** | **Females (N=3)** |
| Cₘₐₓ (µg/g) | 0.189 ± 0.0202 | 0.168 ± 0.0157 | 0.574 ± 0.1230 | 0.635 ± 0.1028 |
| Tₘₐₓ (hr) | 1.0 | 0.5 | 1.0 ± 0 | 0.8 ± 0.3 |
| AUC (µg·hr/mL) | 0.498 ± 0.0176 | 0.506 ± 0.0633 | 2.03 ± 0.406 | 1.92 ± 0.150 |
| t_{½} (hr) | 1.1 ± 0.04 | 1.4 ± 0.17 | 2.1 ± 0.14 | 1.8 ± 0.04 |

| | | | | |
|---|---|---|---|---|
| 1. Per time point | | | | |

When administered orally, fampridine is completely absorbed from the gastrointestinal tract. The absolute bioavailability of two formulations of IR tablets was reported to be 95% (Uges et al., 1982). Absolute bioavailability of Fampridine-SR tablets has not been assessed, but relative bioavailability (as compared to an aqueous oral solution) is 95% Absorption is rapid unless administered in a modified matrix. When a single Fampridine-SR tablet 10 mg dose is administered to healthy volunteers while in a fasted state, mean peak concentrations ranging in different studies from 17.3 ng/mL to 21.6 ng/mL occurred 3 to 4 hours post-administration (Tₘₐₓ). In comparison, the Cₘₐₓ achieved with the same 10 mg dose of a fampridine oral solution was 42.7 ng/mL which occurred approximately 1.1 hours after dose administration. Exposure increases proportionally with dose, and steady state maximum concentrations are approximately 29-37% higher than for single doses.

Table 2 illustrates the dose proportionality of 10 mg and 25 mg single doses and the relative bioequivalence of a solid oral dosage form and oral solution.

**Table 2: Relative Bioavailability/Bioequivalence Summary Study Results Conducted in Healthy Adult Volunteers (N=26 with Data)**

| **Parameter** | **Dose** | | | **10 mg *vs.* solution** | | **10 mg *vs*. 25 mg (dose-adjusted)** | |
|---|---|---|---|---|---|---|---|
| | **Fampridine SR Tablet Dose** | | **Buffered Solution (0.83 mg**/**mL**) | **Ratio of Geometric Means*** | **90% CI** | **Ratio of Geometric Means*** | **90% CI** |
| | **10 mg** | **25 mg** | **10 mg** | | | | |
| ln-Cₘₐₓ | 2.91 | 3.77 | 3.73 | 43.6 | 41.07-46.35 | 104.3 | 98.07-110.88 |
| ln-AUC₍₀₋ₜ₎ | 5.21 | 6.09 | 5.35 | 86.7 | 80.60-93.26 | 102.1 | 94.96-109.99 |
| ln-AUC_{(0- inf)} | 5.37 | 6.17 | 5.42 | 94.7 | 88.23-101.55 | 110.9 | 103.20-119.25 |

The dose proportionality of exposure following single doses of Fampridine-SR is illustrated in Table 3. The pharmacokinetic disposition following of multiple doses of Fampridine-SR is illustrated in Table 4.

**Table 3: Dose-Normalized Pharmacokinetic Parameter Values (Mean ± SEM) Following Single Oral Administration of Fampridine-SR Tablets to Patients with MS**

| | **Dose (mg)** | | | |
|---|---|---|---|---|
| **Parameter** | **5 (n=24)** | **10 (n=24)** | **15 (n=24)** | **20 (n=23)** |
| Cₘₐₓ-norm* (ng/mL) | 13.1 ± 0.6 | 12.6 ± 0.7 | 12.3 ± 0.7 | 12.3 ± 0.8 |
| Tₘₐₓ (hours) | 3.9 ± 0.2 | 3.9 ± 0.3 | 3.6 ± 0.3 | 3.6 ± 0.3 |
| AUC-norm* (ng·hr/mL) | 122.1 ± 9.4 | 122.1 ± 9.4 | 131.5 ± 7.4 | 127.8 ± 6.9 |
| t_{½} (hours) | 5.8 ± 0.5 | 5.6 ± 0.4 | 5.5 ± 0.4 | 5.1 ± 0.3 |
| Cl/F (mL/min) | 619.8 ± 36.2 | 641.4 ± 39.1 | 632.4 ± 39.0 | 653.9 ± 37.1 |

| | | | | |
|---|---|---|---|---|
| * Normalized to a 5 mg dose. | | | | |

**Table 4: Pharmacokinetic Parameter Values (Mean and 95% CI) Following Multiple Oral Doses of Fampridine-SR Tablets (40 mg/day, 20 mg b.i.d.) in 20 Patients with MS**

| | **Parameter** | | | | |
|---|---|---|---|---|---|
| **Day** | **Cₘₐₓ (ng/mL)** | **Tₘₐₓ (hours)** | **AUC₍₀₋₁₂₎ (ng·hr/mL)** | **t_{½} (hours)** | **Cl/F (mL/min)** |
| Day 1 | 48.6 (42.0, 55.3) | 3.8 (3.2, 4.3) | NE | NE | NE |
| Day 7/8 | 66.7 (57.5, 76.0) | 3.3 (2.8, 3.9) | 531 (452,610) | NE | 700 (557, 844) |
| Day 14/15 | 62.6 (55.7, 69.4) | 3.3 (2.6, 3.9) | 499 (446, 552) | 5.8 (5.0, 6.6) | 703 (621, 786) |

| | | | | | |
|---|---|---|---|---|---|
| NE = Not evaluable | | | | | |

### Distribution

The volume of distribution at steady state (V_{dss}) in rats has been reported to approximate total body volume (not adjusted for bioavailability). Following administration of a single p.o. dose of 4-aminopyridine (2 mg/kg) to male and female rats, V_{dss} is 13% lower in females than in males (1094.4 mL in males versus 947.5 mL in females); however, the difference is not statistically significant. Furthermore, when adjusted for body weight differences, there is no difference between males and females (2%).

In a single-dose study, rats were administered ¹⁴C-labeled 4-aminopyridine (1 mg/kg) p.o. Three animals per time point were sacrificed 1, 3, 8, and 24 hours post-dose. Blood was collected and tissues were excised for determination of radioactivity. One hour post-dose, at a time approximately corresponding to the peak plasma concentration, radioactivity was detected in all tissues collected. The amounts represented small percentages of the dose; however, only 58.3% of the dose was accounted for in total. The highest concentrations were in the liver (2.6%), kidney (1.6%), and blood (0.7%); 51% of the radioactivity was in the carcass (primarily the gastrointestinal tract and musculoskeletal system). The half-life of elimination from tissues ranged from 1.1 to 2.0 hours. By 3 hours post-dose, the amount of radioactivity detected in all tissues was negligible (with the exception of the carcass, which contained 15.4% of the radioactive dose).

An in vitro study was conducted to assess plasma protein binding in rat and dog plasma. 4-Aminopyridine concentrations of 5, 50, or 500 ng/mL were used. 4-Aminopyridine was largely unbound and had a high free drug fraction at all three concentrations tested. After a 4-hour dialysis period, the mean percent of free drug ranged from 73 to 94% in rat plasma and 88 to 97% in dog plasma.

Specific studies describing the distribution of 4-aminopyridine across the blood:brain barrier, across the placenta, or into milk have not been identified. However, in the rat, ¹⁴C-labeled 4-aminopyridine was detected in the cerebrum and cerebellum at tissue-to-blood ratios of 3.07 and 1.48, respectively, indicating that 4-aminopyridine crosses the blood brain barrier following an oral dose. 4-aminopyridine is eliminated from the brain at a similar rate as from the blood. Specifically, the elimination half-lives of 4-aminopyridine from brain tissues (cerebellum and cerebrum) and the blood are similar (1.24, 1.63, and 1.21 hours, respectively).

Fampridine is largely unbound to plasma proteins (97 to 99%). Administration of a single 20 mg intravenous dose, mean Vd is 2.6 L/kg, greatly exceeding total body water (Uges et al., 1982), similar to values calculated in healthy volunteers and patients with SCI who receive Fampridine-SR tablets. The plasma concentration-time profile is one of two or three compartments with a rapid initial distribution phase. Measurable levels are present in the saliva.

### Toxicology

In single- and repeated-dose toxicity studies, the dosing regimen greatly affected the rate of mortality and incidence of clinical signs in all species studied (with the possible exception of the mouse). In general, higher mortality rates and greater incidences of adverse clinical signs were noted when 4-aminopyridine was administered in a single large dose as compared to when the same total dose was given as two, three, or four equally divided sub-doses. Toxic responses to orally administered 4-aminopyridine were rapid in onset, most often occurring within the first 2 hours post-dose.

Clinical signs evident after large single doses or repeated lower doses were similar in all species studied and included tremors, convulsions, ataxia, dyspnea, dilated pupils, prostration, abnormal vocalization, increased respiration, excess salivation, gait abnormalities, and hyper- and hypo-excitability. These clinical signs were not unexpected and represent exaggerated pharmacology of 4-aminopyridine.

In controlled clinical studies involving the use of fampridine, the most frequent adverse events by body system occurred in the nervous system, "body as a whole", and digestive system. Dizziness, insomnia, paresthesia, pain, headache and asthenia are the most common nervous system adverse events, and nausea is the most frequently reported event in the digestive system category.

The most frequent treatment-related adverse events that have been reported with fampridine-SR, in MS patients as well as other populations including spinal cord injury, may be broadly categorized as excitatory effects in the nervous system, which would be consistent with the potassium channel blocking activity of the compound. These adverse events include dizziness, paresthesias, insomnia, balance disorders, anxiety, confusion and seizure. While an increased incidence of such events appears to be moderately dose-related, the susceptibility of individuals is quite variable. The potential for lowering seizure threshold in people with MS appears to be more significant than for people with spinal cord injury, which may result from interaction of the channel-blocking properties of the drug with MS brain pathology in certain individuals.

Also disclosed are methods of using 4-aminopyridine for treating multiple sclerosis and one or more of the symptoms thereof; MS-related symptoms treated in accordance with the disclosure comprise neuro-cognitive and/or neuro-psychiatric disorders. Accordingly, also disclosed are the following:

A method of effectively treating multiple sclerosis in a patient over a short-term, initial, or non-chronic time period: comprising administering a therapeutically effective amount of 4-aminopyridine to said patient; in certain embodiments the period is 1, 2, 3, 4, 5, 6, 7, 8, 9, 10, 11, 12, 13, 14, 15 day(s); 1, 2, 3, 4, 5, 6, 7, 8, 9, 10, 11, 12, 13, 14, 15 weeks; 1, 2, 3, or 4 months. It is understood that one can continue beyond such period.

A method of effectively treating multiple sclerosis in a patient over a chronic time period: comprising administering a therapeutically effective amount of 4-aminopyridine to said patient for an extended period of time. Also disclosed is a method of durably treating multiple sclerosis in a patient, comprising: administering a therapeutically effective amount of 4-aminopyridine to said patient for an extended period of time. Also disclosed is a method wherein the extended period is at least or is more than: 10, 11, 12, 13, 14, 15, 16, 17, 18, 19, 20, 21 or 22 weeks; 3, 4, 5, 6, 7, 8, 9, 10, 11, 12, 13, 14, 15, 16, 17, or 18 months; or 1, 2, 3, 4, 5, 6, or greater than 5 years.

Also disclosed is a method for maintaining improvement of a symptom of multiple sclerosis in a patient, said method comprising: administering a therapeutically effective amount of 4-aminopyridine to said patient after previously achieving an improvement of a symptom of multiple sclerosis in said patient during administration of 4-aminopyridine.

Also disclosed is a method for maintaining improvements in one or more of neuro-cognitive, neuro-psychiatric, cognitive and mental disorders, mood, cognition, and/or psychiatric/psychological factors, in a patient with multiple sclerosis comprising administering a therapeutically effective amount of 4-aminopyridine to said patient over an extended period of time.

Also disclosed is a method for achieving sustained improvement in a patient with multiple sclerosis in a parameter selected from, e.g., any of the following: neuro-cognitive, neuro-psychiatric, cognitive and mental disorders, mood, cognition, and/or psychiatric/psychological factors; the method comprising continuing administration a therapeutically effective amount of 4-aminopyridine to said patient over an extended period of time.

Also disclosed is a method wherein said therapeutically effective amount of 4-aminopyridine is in a range of about 10 milligrams in a sustained release composition twice daily. Also disclosed is a method wherein said therapeutically effective amount of 4-aminopyridine is in a range of about 20 milligrams in a sustained release composition daily.

Also disclosed is a method wherein said therapeutically effective amount of 4-aminopyridine achieves a Cₘᵢₙₛₛ of at least or more than: 11, 12, 13, 14, 15, 16, 17, 18,19 or 20 ng/ml. Also disclosed is a method wherein said therapeutically effective amount of 4-aminopyridine achieves an average Cₘᵢₙₛₛ of at least or more than: 11, 12, 13, 14, 15, 16, 17, 18, 19 or 20 ng/ml. Also disclosed, an amount of drug is given to an individual patient (e.g., a dose amount) wherein that dose amount corresponds to an amount that when administered to a normative or reference population obtains an average Cₘᵢₙₛₛ of at least or more than: 11, 12, 13, 14, 15, 16, 17, 18, 19 or 20 ng/ml. Fluid or tissue levels (e.g., Cₘᵢₙₛₛ, Cₘₐₓₛₛ, Cₐᵥₛₛ) in reference population can be referred to as normative values. Also disclosed is a method wherein said therapeutically effective amount of 4-aminopyridine achieves a Cₘᵢₙₛₛ in a range of about 13 to 15 ng/ml. Also disclosed is a method wherein said therapeutically effective amount of 4-aminopyridine achieves a Cₘᵢₙₛₛ in a range of 20 ng/ml. Also disclosed, a Cₘᵢₙₛₛ in a range of 20 ng/ml achieves a Cₘᵢₙₛₛ of about 20 ng/ml. Also disclosed is a method wherein said therapeutically effective amount of 4-aminopyridine achieves a Cₘᵢₙₛₛ of about 20 ng/ml; also disclosed, a Cₘᵢₙₛₛ of about 20 ng/ml comprises a lower limit value of from 11, 12, 13, 14, 15, 16, 17, 18, 19, or 20 ng/ml, and an upper limit value of 20, 21, 22, 23, 24, 25, 26, or 27 ng/ml.

Also disclosed is a method wherein said therapeutically effective amount of 4-aminopyridine achieves a Cₘₐₓₛₛ of any of the following, or less than any of the following: 34, 33, 32, 31, 30, 29, 28, 27,26, 25, 24, 23, 22, 21, or 20 ng/ml. Also disclosed is a method wherein said therapeutically effective amount of 4-aminopyridine achieves an average Cₘₐₓₛₛ of any of the following, or less than any of the following: 34, 33, 32, 31, 30, 29, 28, 27, 26, 25, 24, 23, 22, 21, or 20 ng/ml. Also disclosed, an amount of drug is given to an individual patient (e.g., a dose amount) wherein that dose amount corresponds to an amount that when administered to a normative or reference population obtains an average Cₘₐₓₛₛ of the following, or less than the following: 34, 33, 32, 31, 30, 29, 28, 27, 26, 25, 24, 23, 22, 21, or 20 ng/ml. Fluid or tissue levels (e.g., Cₘᵢₙₛₛ, Cₘₐₓₛₛ, Cₐᵥₛₛ) in reference population can be referred to as normative values. Also disclosed is a method wherein said therapeutically effective amount of 4-aminopyridine achieves a Cₘₐₓₛₛ in a range of about 25 to 35 ng/ml. Also disclosed is a method wherein said therapeutically effective amount of 4-aminopyridine achieves a Cₘₐₓₛₛ in a range of 30 ng/ml. Also disclosed, a Cₘₐₓₛₛ in a range of 30 ng/ml achieves a Cₘₐₓₛₛ of about 30 ng/ml. Also disclosed is a method wherein said therapeutically effective amount of 4-aminopyridine achieves a Cₘₐₓₛₛ in a range that comprises a lower limit value of from 25, 26, 27, 28, 29, 30 ng/ml, and an upper limit value of 25,26,27,28,29,30,31,32,33,34 or 35 ng/ml.

Also disclosed is a composition as substantially described herein. Also disclosed is a method as substantially described herein.

Also disclosed is a method of treating one or more symptoms of multiple sclerosis as substantially described herein; these symptoms can comprise any one or more of: neuro-cognitive, neuro-psychiatric, cognitive and mental disorders, mood, cognition, and/or psychiatric/psychological factors.

Disclosed herein, there is a method of treating multiple sclerosis in a patient comprising: administering a therapeutically effective amount of 4-aminopyridine to said patient such that a Cₘᵢₙₛₛ in a range of 12 ng/ml to 20 ng/ml is obtained. Also disclosed is a method wherein said therapeutically effective amount of 4-aminopyridine achieves a Cₘᵢₙₛₛ in a range of 20 ng/ml. Also disclosed, a Cₘᵢₙₛₛ in a range of 20 ng/ml achieves a Cₘᵢₙₛₛ of about 20 ng/ml. Also disclosed is a method wherein said therapeutically effective amount of 4-aminopyridine achieves a Cₘᵢₙₛₛ of about 20 ng/ml; also disclosed, a Cₘᵢₙₛₛ of about 20 ng/ml comprises a lower limit value of from 11, 12, 13, 14, 15, 16, 17, 18, 19, or 20 ng/ml, and an upper limit value of any of 20, 21, 22, 23, 24, 25, 26, or 27 ng/ml. Also disclosed is a method for treating multiple sclerosis in a patient comprising: administering a therapeutically effective amount of 4-aminopyridine to said patient such that a Cₘᵢₙₛₛ of at least or more than any of 10, 11, 12, 13, 14, 15, 16, 17, 18, 19 or 20 ng/ml is obtained. Also disclosed is a method for treating multiple sclerosis in a patient comprising: administering a therapeutically effective amount of 4-aminopyridine to said patient such that a Cₘᵢₙₛₛ in a range of at least 12 ng/ml to 15 ng/ml is obtained. Also disclosed is a method for treating multiple sclerosis in a patient comprising: administering a therapeutically effective amount of 4-aminopyridine to said patient such that a Cₘᵢₙₛₛ in a range of at least 13 ng/ml to 15 ng/ml is obtained.

In another embodiment, a use in accordance with the invention comprises a therapeutically effective amount of 4-aminopyridine is administered weekly, every three days, every other day, once daily, twice daily or thrice daily. In another embodiment, a use wherein said therapeutically effective amount of 4-aminopyridine is about 5 milligrams in a sustained release composition twice daily. In another embodiment, a use wherein said therapeutically effective amount of 4-aminopyridine is about 7.5 milligrams in a sustained release composition twice daily. In another embodiment, a use wherein said therapeutically effective amount of 4-aminopyridine is about 10 milligrams in a sustained release composition twice daily. In another embodiment, a use wherein said therapeutically effective amount of 4-aminopyridine is about 12.5 milligrams in a sustained release composition twice daily. In another embodiment, a use wherein said therapeutically effective amount of 4-aminopyridine is about 15 milligrams in a sustained release composition twice daily. In another embodiment, a use wherein said therapeutically effective amount of 4-aminopyridine is about 17.5 milligrams in a sustained release composition twice daily.

In another embodiment, a use in accordance with the invention comprises a therapeutically effective amount of 4-aminopyridine is about 20 milligrams in a sustained release composition once-daily. In another embodiment, a use wherein said therapeutically effective amount of 4-aminopyridine is about any of 10, 11, 12, 12.5, 13, 14, 15, 16, 17, 17.5, 18, 19, 20, 20.5, 21, 21.5, 22, 22.5, 23, 23.5, 24, 24.5, 25, 25.5, 26, 26.5, 27.5 milligrams in a sustained release composition once daily.

In another embodiment, a use in accordance with the invention comprises a therapeutically effective amount of 4-aminopyridine in a total daily amount of about 10, 11, 12, 12.5, 13, 14, 15, 16, 17, 17.5, 18, 19, 20, 20.5, 21, 21.5, 22, 22.5, 23, 23.5, 24, 24.5, 25, 25.5, 26, 26.5, 27.5, 28, 29, 30, 31, 32, 33, 34, 35 milligrams in a sustained release composition. Another exemplary embodiment comprises twice daily administration where 15 milligrams in a sustained release composition is administered in the morning; and 10 milligrams in a sustained release composition is administered in the evening. Another exemplary embodiment comprises twice daily administration where 12.5 milligrams in a sustained release composition is administered in the morning; and 7.5 milligrams in a sustained release composition is administered in the evening.

Also disclosed is a method wherein said therapeutically effective amount of 4-aminopyridine achieves an average Cₘᵢₙₛₛ of at least or more than any of: 11, 12, 13, 14, 15, 16, 17, 18, 19 or 20 ng/ml. In one embodiment, an amount of drug is given to an individual patient (e.g., a dose amount) wherein that dose amount is corresponds to a dose that when administered to a normative or reference population obtains an average Cₘᵢₙₛₛ of at least or more than any of: 10, 11, 12, 13, 14, 15, 16, 17, 18, 19 or 20 ng/ml; the plasma levels (e.g., Cₘᵢₙₛₛ, Cₘₐₓₛₛ, Cₐᵥₛₛ) in reference population can be referred to as a normative values.

Also disclosed is a method wherein said therapeutically effective amount of 4-aminopyridine achieves an average Cₘₐₓₛₛ of, or less than any of: 35, 34, 33, 32, 31, 30, 29, 28, 27, 26, 25, 24, 23, 22, 21, or 20 ng/ml. In one embodiment, an amount of drug is given to an individual patient (e.g., a dose amount) wherein that dose amount is corresponds to a dose that when administered to a normative or reference population obtains an average Cₘₐₓₛₛ of, or less than any of: 35, 34, 33, 32, 31, 30, 29, 28, 27, 26, 25, 24, 23, 22, 21, or 20 ng/ml; the plasma levels (e.g., Cₘᵢₙₛₛ, Cₘₐₓₛₛ, Cₐᵥₛₛ) in reference population can be referred to as a normative values.

Also disclosed is a composition as substantially described herein. Also disclosed is a method as substantially described herein. Also disclosed is a method of treating one or more symptoms of multiple sclerosis as substantially described herein; these symptoms can comprise any one or more of: neuro-cognitive, neuro-psychiatric, cognitive and mental disorders, mood, cognition, and/or psychiatric/psychological factors.

In certain embodiments, the therapeutically effective amount of 4-aminopyridine is a stable or constant or consistent or unchanging or unwavering or unaltered dosing regimen that comprises a therapeutically effective amount of 4-aminopyridine that is administered at a uniform pattern (e.g., a milligram amount or particular milligram amount at particular times of day, e.g. there may be a higher dose in the morning and a lower dose in the evening or vice versa) and on a uniform schedule (e.g., twice daily), wherein no changes of the dose amount or schedule occurs during the stable or constant or consistent or unchanging or unwavering dosing regimen. As used herein, the terms "stable" or "constant" or "consistent" or "unchanging" or "unwavering" or "unaltered" are synonyms unless the context clearly indicates otherwise. It is to be understood that, e.g., occasional patient noncompliance or deviation from an otherwise stable, constant, consistent, unchanging, unwavering, or unaltered course of treatment is within the definition of such treatment. In certain embodiments, no titration (whether an increase or decrease) of the dose (e.g., milligram amount) of 4-aminopyridine occurs during the entirety of the stable dosing regimen. In certain embodiments, the therapeutically effective amount of 4-aminopyridine is 10 milligrams in a sustained release composition. In certain embodiments, the sustained release composition may be administered twice daily. In certain embodiments, the sustained release composition may be administered once daily. These methods can also comprise administering the 4-aminopyridine at or to a therapeutic level (such as Cₘᵢₙₛₛ) or range (such as a Cₘᵢₙₛₛ range) in accordance with the present invention.

Methods disclosed herein allow for maintaining improvement of a symptom, parameter, characteristic, value, finding or manifestation of multiple sclerosis in a patient, where such symptom, parameter, characteristic, value, finding or manifestation was previously effectively addressed by 4-aminopyridine, by administering a therapeutically effective amount of 4-aminopyridine to said patient (after previously achieving an improvement of such symptom, parameter, characteristic, value, finding or manifestation). In one embodiment, the parameter that is maintained is neuro-cognitive and/or neuro-psychiatric ability(s). The previous period of efficacy can be 10, 11, 12, 13, 14, 15, 16, 17 or 18 weeks; 3, 4, 5, 6, 7, 8, 9, 10, 11, 12 or 13 months; 1, 2, 3, 4, 5, 6, 7, 8, 9, 10, or more than 10 years.

### Mental Status and Cognition:

Neuro-cognitive dysfunction is a significant problem in many neurological conditions, such as demyelinating conditions, traumatic brain injury, cerebral palsy or post-radiation encephalopathy. Previously, aminopyridines have been explored for their effects on cognition in patients with neurologic conditions. In particular, aminopyridines have been evaluated in conditions such as MS that involve demyelinating neuropathological processes and Alzheimer's Disease that involves different neuropathological mechanisms. The study results to-date have not been definitively positive or negative.

Cognitive dysfunction, for example, is one of the most frequent causes of disability in MS. (see, e.g., Rao SM, et al. Neurology. 1991;41:692-696). Results from fMRI studies on cognition have shown a significant delay in processing of information due to areas of demyelination (T2 lesion volume) disrupting processing from one area of the brain to another. (Bobholz JA, et al. Neurology. 2006;67:1640-1645) However, a prior study with 4-aminopyridine did not show benefit of cognitive function in MS patients. (Smits RC, et al. Neurology. 1994;44:1701-1705).

Disclosed for the first time herein is a regimen that facilitates improvement and/or stabilization in one or more brain functions by utilizing an aminopyridine, e.g., 4AP, fampridine or Fampridine-SR in MS. In particular, a dosing regimen is disclosed that is found to elicit one or more improvement(s) in neuro-cognitive function or a reduction in related neuro-psychiatric complications commonly observed in patients; related neuro-psychiatric conditions include such conditions such as depression, altered libido, euphoria or fatigue.

### Patient identification or selection:

In a preferred embodiment of the disclosure, patients are provided, identified or diagnosed who have at least one of: a demyelinating condition, traumatic brain injury, cerebral palsy, or post-radiation encephalopathy; and which patient(s) also have an impairment or alteration in brain function such as decreased neuro-cognitive ability(s). The need to improve neuro-cognitive (and related neuro-psychiatric issues) function is an area of particular interest, especially for patients with demyelinating conditions such as MS. The alteration in brain function can also include a disease-related neuro-psychiatric conditions(s) such as depression, altered libido, euphoria or fatigue. Patients having MS are then administered a composition and a dosing regimen in accordance with the present invention.

### Dosing Regimens:

In a simplified description of neuromuscular connections comprise: a cortical motor neuron, a spinal motor neuron and a muscle. In contrast, there is no such simple embodiment for the anatomy involved in neuro-cognitive functions, and the various aspects thereof. Neuro-cognitive functions, and related neuro-psychiatric conditions, are more diffuse and integrated phenomena. Consequently, regimens previously employed for, e.g., neuromuscular therapies are not dispositive of therapeutic dosing for neuro-cognitive functions or related neuro-psychiatric conditions.

In view of the complex, integrated nature of neuro-cognitive and related neuro-psychiatric phenomena, it is disclosed herein that there is particular merit and value to manage the range of steady state aminopyridine concentration values,e.g., in the plasma, and particularly inside the blood:brain barrier, in CNS tissue and/or in the CSF. In a preferred embodiment, the requisite control is achieved by prescribing, dosing, administering, consuming, and/or use of a composition such as an aminopyridine, fampridine, or Fampridine-SR. In one embodiment, the Fampridine-SR is dosed on a bid or every 12-hour dosing protocol. In particular embodiments, doses of 15, 12.5, 10, 7.5, 5 or 2.5 mg of Fampridine-SR bid are embodiments of the invention.

It is disclosed for the first time herein that certain side effects that come about upon administration of aminopyridines, such as fampridine, are particularly problematic when the primary effect desired by the drug is an improvement or stabilization in a neuro-cognitive or related neuro-psychiatric parameter. For example, aminopyridines elicit certain neurological-related side effects such as tremors, anxiety, confusion, seizure, convulsions, ataxia, hyper-excitability, hypo-excitability, seizure, insomnia, headache, asthenia, dizziness, balance disorder, and/or paresthesias; these side effects tend to be more common as the dose increases. These effects, although not desired, are not necessarily directly contrary to, e.g., a desired neuromuscular effect. When, however the desired effect is a neuro-cognitive or neuro-psychiatric one, it is now appreciated that the management of these undesired effects is particularly important to an efficacious outcome. There is negative impact from side effects with regard to desired neuro-cognitive/neuro-psychiatric benefits elicited from dosing with aminopyridines, such as fampridine. Many of the side effects when dosing aminopyridines are confounding variables when evaluating the efficacy of these drugs in the context of neuro-cognitive and related neuro-psychiatric disorders. The uses of the invention are particularly important in curtailing these side effects when treating neuro-cognitive/psychiatric problems in patients of the invention; confounding variable(s) in achieving efficacy are avoided in accordance with the present invention.

In a preferred embodiment of the present invention, a patient in need of treatment, which need is as appreciated by one of ordinary skill in the art, is provided with Fampridine-SR. The patient is instructed to take the drug twice daily. Generally, the patient is instructed to take the drug in a dose in a range between 2.0 and 13.0 mg of Fampridine-SR bid. Often the dose is selected from 2.5, 5.0, 7.5, 10 or 12.5 mg Fampridine-SR bid. In a preferred embodiment, the amount of Fampridine-SR is 10 mg bid. In another embodiment a formulation comprising 4AP is provided to a patient in an amount that was found to achieve the plasma concentration elicited by steady state administration of 4AP in a normative population.

In an alternative dosing embodiment, a sufficient amount of aminopyridine, such as fampridine, is provided such that it elicits the steady state levels that are within the range obtained by use of Fampridine-SR in accordance with the invention. In one embodiment these steady state values are delimited by a maximum concentration at steady state (Cₘₐₓₛₛ) and minimum concentration at steady state (Cₘᵢₙₛₛ). The steady state values can be plasma levels, levels on the brain side of the blood:brain barrier, or levels in the brain tissue or in the CSF. Preferably, these are plasma levels.

In another embodiment, a sufficient amount of an aminopyridine, such as 4-aminopyridine, is provided that it elicits the steady state levels that differ not more than about 15, 14, 13, 12, 11, 10, 9, 8, 7, 6, 5, 4.5, 4, 3.5, 3, 2.5, 2, 1.5 or 1% from (Cₘₐₓₛₛ) and (Cₘᵢₙₛₛ) obtained by use of Fampridine-SR in accordance with the invention. The steady state values can be plasma levels, levels on the brain side of the blood:brain barrier, or levels in the CSF. In a particular embodiment these are plasma levels.

In another embodiment, a sufficient amount of an aminopyridine, such as 4-aminopyridine, is provided that it elicits the steady state levels that differ not more than about 15, 14, 13, 12, 11, 10, 9, 8, 7, 6, 5, 4, 3, 2, or 1% from the average steady state level (Cₐᵥₛₛ) obtained by use of Fampridine-SR. The steady state values can be plasma levels, levels on the brain side of the blood:brain barrier, or levels in the CSF. Preferably, these are plasma levels.

Moreover, in view of the complex, integrated nature of neuro-cognitive and related neuro-psychiatric phenomena, it is disclosed herein that for certain patients there is particular merit and value to treat in accordance with the invention for a sufficient time such that changes in mental status can resolve. It is presently understood that this time frame is the byproduct of the integrated nature of higher level brain function, and the complex accommodations made by various components of the relevant anatomy to administered therapeutics such as aminopyridines. Thus, in certain embodiments, treatment in accordance with the invention takes place for at least 1, 2, 3, 4, 5, 6, 7, 8, 9, 10 , 11, 12, or more than 12 weeks. In certain embodiments, treatment in accordance with the invention takes place for at least 1, 2, 3, 4, 5, 6, 7, 8, 9, 10, 11, 12, 13, 14, 15, 16, 17, 18 or more than 18 months.

The disclosure relates to methods of using 4-aminopyridine for treating multiple sclerosis, and in particular neuro-cognitive/neuro-psychiatric effects/symptoms thereof. Such aspects of this disclosure include the following:

A method of effectively treating multiple sclerosis (e.g., neuro-cognitive/neuro-psychiatric effects/symptoms) in a patient over a chronic time period: comprising administering a therapeutically effective amount of 4-aminopyridine to said patient for an extended period of time. Also disclosed is a method of durably treating multiple sclerosis in a patient: comprising administering a therapeutically effective amount of 4-aminopyridine to said patient for an extended period of time. Also disclosed is a method wherein the extended period is at least or is more than: 1, 2, 3, 4, 5, 6, 7, 8, 9, 10, 11, 12, 13, 14, 15, 16, 17, 18, 19, 20, 21 or 22 weeks; 1, 2, 3, 4, 5, 6, 7, 8, 9, 10, 11, 12, 13, 14, 15, 16, 17, or 18 months; or 1, 2, 3, 4, 5, 6, or greater than 5 years.

Also disclosed is a method for maintaining improvement of a symptom of multiple sclerosis in a patient (such as a neuro-cognitive/neuro-psychiatric improvement), said method comprising: administering a therapeutically effective amount of 4-aminopyridine to said patient after previously achieving an improvement of a symptom of multiple sclerosis in said patient during administration of 4-aminopyridine. Also disclosed is a method for maintaining improved neuro-cognitive/neuro-psychiatric ability in a patient with multiple sclerosis comprising administering a therapeutically effective amount of 4-aminopyridine to said patient over an extended period of time. Also disclosed is a method for achieving sustained improvement in neuro-cognitive or neuro-psychiatric ability in a patient with multiple sclerosis comprising continuing administration a therapeutically effective amount of 4-aminopyridine to said patient over an extended period of time. Also disclosed is a method wherein said therapeutically effective amount of 4-aminopyridine is 10 milligrams in a sustained release composition twice daily. Also disclosed is a method wherein said therapeutically effective amount of 4-aminopyridine achieves a Cₘᵢₙₛₛ of at least or more than: 11, 12, 13, 14, 15, 16, 17, 18, 19 or 20 ng/ml. Also disclosed is a method wherein said therapeutically effective amount of 4-aminopyridine achieves an average Cₘᵢₙₛₛ of at least or more than: 11, 12, 13, 14, 15, 16, 17, 18, 19 or 20 ng/ml. Also disclosed, an amount of drug is given to an individual patient (e.g., a dose amount) wherein that dose amount corresponds to an amount that when administered to a normative or reference population obtains an average Cₘᵢₙₛₛ of at least or more than: 11, 12, 13, 14, 15, 16, 17, 18, 19 or 20 ng/ml. Fluid or tissue levels (e.g., Cₘᵢₙₛₛ, Cₘₐₓₛₛ, Cₐᵥₛₛ) in reference population can be referred to as normative values. Also disclosed is a method wherein said therapeutically effective amount of 4-aminopyridine achieves a Cₘᵢₙₛₛ in a range of about 13 to 15 ng/ml. Also disclosed is a method wherein said therapeutically effective amount of 4-aminopyridine achieves a Cₘᵢₙₛₛ in a range of about 10 to 17 ng/ml. Also disclosed is a method wherein said therapeutically effective amount of 4-aminopyridine achieves a Cₘᵢₙₛₛ in a range of about 12 to 16 ng/ml. Also disclosed is a method wherein said therapeutically effective amount of 4-aminopyridine achieves a Cₘᵢₙₛₛ in a range of about 12 to 22 ng/ml. Also disclosed is a method wherein said therapeutically effective amount of 4-aminopyridine achieves a Cₘᵢₙₛₛ in a range of 20 ng/ml. Also disclosed, a Cₘᵢₙₛₛ in a range of 20 ng/ml achieves a Cₘᵢₙₛₛ of about 20 ng/ml. Also disclosed is a method wherein said therapeutically effective amount of 4-aminopyridine achieves a Cₘᵢₙₛₛ of about 20 ng/ml; also disclosed, a Cₘᵢₙₛₛ of about 20 ng/ml comprises a lower limit value of 10, 11, 12, 13, 14, 15, 16, 17, 18, 19, or 20 ng/ml, and an upper limit value of 20, 21, 22, 23, 24, 25, 26, 28, 29 or 30 ng/ml. Disclosed herein is a composition as substantially described herein. Disclosed herein is a method as substantially described herein. Also disclosed is a method of increasing neuro-cognitive ability, neuro-psychiatric ability, mental status ability or cognitive ability as substantially described herein. Also disclosed is a method of treating the symptoms of multiple sclerosis as substantially described herein.

Also disclosed, there is a method of treating multiple sclerosis, such as neuro-cognitive or neuro-psychiatric impairments thereof, in a patient comprising: administering a therapeutically effective amount of 4-aminopyridine to said patient such that a Cₘᵢₙₛₛ in a range of 11 ng/ml to 20 ng/ml, 10 ng/ml to 18 ng/ml, 12 ng/ml to 17 ng/ml, or, 11 ng/ml to 21 ng/ml is obtained. Also disclosed is a method wherein said therapeutically effective amount of 4-aminopyridine achieves a Cₘᵢₙₛₛ in a range of 20 ng/ml. Also disclosed, a Cₘᵢₙₛₛ in a range of 20 ng/ml achieves a Cₘᵢₙₛₛ of about 20 ng/ml. Also disclosed is a method wherein said therapeutically effective amount of 4-aminopyridine achieves a Cₘᵢₙₛₛ of about 20 ng/ml; also disclosed, a Cₘᵢₙₛₛ of about 20 ng/ml comprises a lower limit value of from 10, 11, 12, 13, 14, 15, 16, 17, 18, 19, or 20 ng/ml, and an upper limit value of 20, 21, 22, 23, 24, 25, 26, or 27 ng/ml. Also disclosed is a method for treating multiple sclerosis in a patient comprising: administering a therapeutically effective amount of 4-aminopyridine to said patient such that a Cₘᵢₙₛₛ of at least or more than 10, 11, 12, 13, 14, 15, 16, 17, 18, 19 or 20 ng/ml is obtained. Also disclosed is a method for treating multiple sclerosis in a patient comprising: administering a therapeutically effective amount of 4-aminopyridine to said patient such that a Cₘᵢₙₛₛ in a range of at least 12 ng/ml, 13 ng/ml or 15 ng/ml is obtained. Also disclosed is a method for treating multiple sclerosis in a patient comprising: administering a therapeutically effective amount of 4-aminopyridine to said patient such that a Cₘᵢₙₛₛ in a range of at least 10 ng/ml to 18 ng/ml, 10 ng/ml to 16 ng/ml, 11 ng/ml to 15 ng/ml, 12 ng/ml to 14 ng/ml, or 13 ng/ml to 15 ng/ml is obtained. Also disclosed is a method wherein said therapeutically effective amount of 4-aminopyridine is administered once daily, twice daily or thrice daily. Also disclosed is a method wherein said therapeutically effective amount of 4-aminopyridine is 10 milligrams in a sustained release composition twice daily. Also disclosed is a method wherein said therapeutically effective amount of 4-aminopyridine achieves an average Cₘᵢₙₛₛ of at least or more than: 10, 11, 12, 13, 14, 15, 16, 17, 18, 19 or 20 ng/ml. Also disclosed, an amount of drug is given to an individual patient (e.g., a dose amount) wherein that dose amount is or corresponds to a dose that when administered to a normative or reference population obtains an average Cₘᵢₙₛₛ of at least or more than: 10, 11, 12, 13, 14, 15, 16, 17, 18, 19 or 20 ng/ml; the plasma levels (e.g., Cₘᵢₙₛₛ, Cₘₐₓₛₛ, Cₐᵥₛₛ) in reference population can be referred to as a normative values.

Also disclosed, there is a method preparing a medicament in accordance with the invention for use in treating multiple sclerosis, such as a neuro-cognitive or neuro-psychiatric impairment(s) thereof, in a patient comprising preparing a medicament for to be administered as a therapeutically effective amount of 4-aminopyridine to said patient such that a Cₘᵢₙₛₛ in a range of 11 ng/ml to 20 ng/ml, 10 ng/ml to 18 ng/ml, 12 ng/ml to 17 ng/ml, or, 11 ng/ml to 21 ng/ml is obtained. Also disclosed, preparing a medicament for use in a method wherein said therapeutically effective amount of 4-aminopyridine achieves a Cₘᵢₙₛₛ in a range of 20 ng/ml. Also disclosed, a Cₘᵢₙₛₛ in a range of 20 ng/ml achieves a Cₘᵢₙₛₛ of about 20 ng/ml. Also disclosed, preparing a medicament for use in a method wherein said therapeutically effective amount of 4-aminopyridine achieves a Cₘᵢₙₛₛ of about 20 ng/ml; also disclosed, a Cₘᵢₙₛₛ of about 20 ng/ml comprises a lower limit value of from 10, 11, 12, 13, 14, 15, 16, 17, 18, 19, or 20 ng/ml, and an upper limit value of 20, 21, 22, 23, 24, 25, 26, or 27 ng/ml. Also disclosed is a method for preparing a medicament for use in treating multiple sclerosis in a patient comprising: administering a therapeutically effective amount of 4-aminopyridine to said patient such that a Cₘᵢₙₛₛ of at least or more than 10, 11, 12, 13, 14, 15, 16, 17, 18, 19 or 20 ng/ml is obtained. Also disclosed is a method for preparing a medicament for use in treating multiple sclerosis in a patient comprising: administering a therapeutically effective amount of 4-aminopyridine to said patient such that a Cₘᵢₙₛₛ in a range of at least 12 ng/ml, 13 ng/ml or 15 ng/ml is obtained. Also disclosed is a method for preparing a medicament for use in treating multiple sclerosis in a patient comprising: administering a therapeutically effective amount of 4-aminopyridine to said patient such that a Cₘᵢₙₛₛ in a range of at least 10 ng/ml to 18 ng/ml, 10 ng/ml to 16 ng/ml, 11 ng/ml to 15 ng/ml, 12 ng/ml to 14 ng/ml, or 13 ng/ml to 15 ng/ml is obtained. Also disclosed is a method of preparing a medicament for use in a method of treating MS wherein said therapeutically effective amount of 4-aminopyridine is administered once daily, twice daily or thrice daily. Also disclosed is a method of preparing a medicament for use in a method wherein said therapeutically effective amount of 4-aminopyridine is 10 milligrams in a sustained release composition twice daily. Also disclosed is a method of preparing a medicament for use in a treatment method wherein said therapeutically effective amount of 4-aminopyridine achieves an average Cₘᵢₙₛₛ of at least or more than: 10, 11, 12, 13, 14, 15, 16, 17, 18, 19 or 20 ng/ml. Also disclosed is a method of preparing a medicament for use in treatment wherein an amount of drug is given to an individual patient (e.g., a dose amount) wherein that dose amount is or corresponds to a dose that when administered to a normative or reference population obtains an average Cₘᵢₙₛₛ of at least or more than: 10, 11, 12, 13, 14, 15, 16, 17, 18, 19 or 20 ng/ml; the plasma levels (e.g., Cₘᵢₙₛₛ, Cₘₐₓₛₛ, Cₐᵥₛₛ) in reference population can be referred to as a normative values.

Also disclosed is a composition as substantially described herein. Also disclosed is a method as substantially described herein. Also disclosed is a method of increasing walking ability as substantially described herein. Also disclosed is a method of treating the symptoms of multiple sclerosis as substantially described herein.

### Formulations and Administration

It is especially advantageous to formulate parenteral compositions in dosage unit form for ease of administration and uniformity of dosage. Dosage unit form as used herein refers to physically discrete units suited as unitary dosages for the subjects to be treated; each unit containing a predetermined quantity of therapeutic compound calculated to produce the desired therapeutic effect in association with the required pharmaceutical carrier. The specification for the dosage unit forms of the invention are dictated by and directly dependent on (a) the unique characteristics of the therapeutic compound and the particular therapeutic effect to be achieved, and (b) the limitations inherent in the art of compounding such a therapeutic compound for the treatment of a selected condition in a patient. Unit dosage forms can be tablets or blister packs. In certain administration protocols a patient may utilize more than a single unit dose at a time, e.g., consume two tablets contained in separate blisters of a blister pack.

Active compounds are administered at a therapeutically effective dosage sufficient to treat a condition associated with a condition in a patient. A "therapeutically effective amount" preferably reduces the amount of symptoms of the condition in the patient by at least about 20%, more preferably by at least about 40%, even more preferably by at least about 60%, and still more preferably by at least about 80% relative to untreated subjects. For example, the efficacy of a compound can be evaluated in an animal model system that may be predictive of efficacy in treating the disease in humans, such as the model systems described herein.

The actual dosage amount of a compound of the present disclosure or composition comprising a compound of the present disclosure administered to a subject may be determined by physical and physiological factors such as age, sex, body weight, severity of condition, the type of disease being treated, previous or concurrent therapeutic interventions, idiopathy of the subject and on the route of administration. These factors may be determined by a skilled artisan. The practitioner responsible for administration will typically determine the concentration of active ingredient(s) in a composition and appropriate dose(s) for the individual subject. The dosage may be adjusted by the individual physician in the event of any complication.

### Combination treatments

The compositions and uses of the present invention may be used in the context of a number of therapeutic or prophylactic applications. In order to increase the effectiveness of a treatment with the compositions of the present invention, e.g., aminopyridines, or to augment the protection of another therapy (second therapy), it may be desirable to combine these compositions and uses with other agents and methods effective in the treatment, amelioration, or prevention of diseases and pathologic conditions, for example, cognitive dysfunctions or impairments or psychiatric dysfunctions or impairments.

Various combinations may be employed; for example, an aminopyridine or derivative or analog thereof, is "A" and the secondary therapy (*e*.*g*., cholinesterase inhibitors such as donepezil, rivastigmine, and galantamine) is "B", nonlimiting combination cycles include:

| | | | | | |
|---|---|---|---|---|---|
| A/B/A | B/AB B/B/A | A/A/B | A/B/B B/A/A | A/B/B/B | B/A/B/B |
| B/B/B/A | B/B/A/B | A/A/B/B | A/B/A/B | A/B/B/A | B/B/A/A |
| B/A/B/A | B/A/AB | A/A/A/B | B/A/A/A | A/B/A/A | A/A/B/A |

Administration of a composition of the present invention to a subject will follow general protocols for the administration described herein, and the general protocols for the administration of a particular secondary therapy will also be followed, taking into account the toxicity, if any, of the treatment. It is expected that the treatment cycles would be repeated as necessary. It also is contemplated that various standard therapies may be applied in combination with the described therapies.

Treatments or secondary therapies for cognitive impairment include: anti-psychotic compounds (e.g., Ziprasidone, Olanzapine, Clozapine, Risperidone, Sertindole, Quetiapine Aripiprazole, Amisuipride, Paliperidone, Bifeprunox); cholinesterase inhibitors (e.g., donepezil, rivastigmine, and galantamine); and nicotinic receptor agonists or antagonists (e.g., varenicline, azaindole-ethylamine derivatives as described in U.S. Pat. No. 5,977,131).

The following examples are given for the purpose of illustrating various embodiments of the invention and are not meant to limit the present invention in any fashion. One skilled in the art will appreciate readily that the present invention is well adapted to carry out the objects and obtain the ends and advantages mentioned, as well as those objects, ends and advantages inherent herein. The present examples, along with the methods described herein are presently representative of preferred embodiments, are exemplary, and are not intended as limitations on the scope of the invention. Changes therein and other uses which are encompassed within the invention as defined by the scope of the claims will occur to those skilled in the art.

### Caveats, Negative Limitations, Exclusions:

Moreover, embodiments of uses in accordance with the invention can specifically exclude embodiments that comprise administering about 10 mg of a sustained release formulation of 4-aminopyridine on a twice daily basis. Embodiments of uses in accordance with the invention can specifically exclude embodiments that comprise administering about 17.5 mg of a sustained release formulation of 4-aminopyridine on a twice daily basis. Embodiments of uses in accordance with the invention can specifically exclude embodiments that comprise administering on a twice daily basis, b.i.d. amounts of a sustained release formulation of 4-aminopyridine in range of about 10-17.5 mg (for clarity this yields a total daily dose of 10-35 mg of 4-aminopyridine).

Embodiments of uses in accordance with the invention can specifically exclude embodiments that comprise administering a total daily amount of a bid formulation of sustained release aminopyridine of about 20 mg. Embodiments of uses in accordance with the invention can specifically exclude embodiments that comprise administering a total daily amount of a bid formulation of sustained release aminopyridine of about 35 mg. Embodiments of uses in accordance with the invention can specifically exclude embodiments that comprise administering a total daily amount of a bid formulation of sustained release aminopyridine in any amount in a range from about 20 mg to about 35 mg of sustained release formulation of 4-aminopyridine.

Embodiments of uses in accordance with the invention can specifically exclude embodiments where, the improved symptom is walking, walking ability, increased walking speed, improved walking speed, or spasticity: Embodiments of uses in accordance with the invention can specifically exclude embodiments where the improved symptom is manifest in the lower extremities. Embodiments of uses in accordance with the invention can specifically exclude embodiments where the improved symptom is spasticity manifest in the lower extremities. Embodiments of uses in accordance with the invention can specifically exclude embodiments where the improved symptom is muscle tone manifest in the lower extremities. Embodiments of uses in accordance with the invention can specifically exclude embodiments where the improved symptom is muscle strength manifest in the lower extremities. In certain embodiments, the improved symptom is not one or more of: walking, walking ability, walking speed, lower extremity muscle tone, lower extremity muscle strength and/or lower extremity spasticity. In certain embodiments, the improved symptom is not cognition. In certain embodiments, the improved symptom is not spasticity.

Accordingly in each of the embodiments set forth herein, further embodiments can comprise a negative limitation or a caveat or proviso that will exclude embodiments that comprise administering about 10 mg of a sustained release formulation of 4-aminopyridine on a twice daily basis; embodiments that comprise administering about 17.5 mg of a sustained release formulation of 4-aminopyridine on a twice daily basis; embodiments that comprise administering any amount in a range from about 10mg to about 17.5 mg of a sustained release formulation of 4-aminopyridine on a twice daily basis; or is not administering a total daily amount of a bid formulation of sustained release aminopyridine of about 20 mg; or is not administering a total daily amount of a bid formulation of sustained release aminopyridine of about 35 mg; or is not administering a total daily amount of a bid formulation of sustained release aminopyridine in any amount in a range of about 20-35 mg sustained release formulation of 4-aminopyridine, or where the improved or treated symptom is not walking, not walking ability, not increased walking speed, not improved walking speed, not cognition and/or not spasticity; where the improved or treated symptom is not manifest in the lower extremities; where the improved or treated symptom is not spasticity manifest in the lower extremities; where the improved or treated symptom is not muscle tone manifest in the lower extremities; where the improved or treated symptom is not muscle strength manifest in the lower extremities.

### EXAMPLES:

### Example 1: Use of Fampridine-SR to Improve Cognitive and/or psychiatric Function

Fampridine has been shown to improve ambulation in large controlled studies in multiple sclerosis (MS) patients. The mechanism of action is proposed to be via block of potassium channels along demyelinated axons. In studies of cognitive dysfunction in MS patients with impaired neuropsychological function, fMRI studies have shown that there is a delay of conduction through demyelinated segments.

Fampridine, when properly dosed, improves neuro-cognitive and/or neuro-psychiatric function; this is believed to occur by improving conduction velocity in interconnecting neurons involved in cognitive and/or psychiatric function in cortical and subcortical regions of the brain.

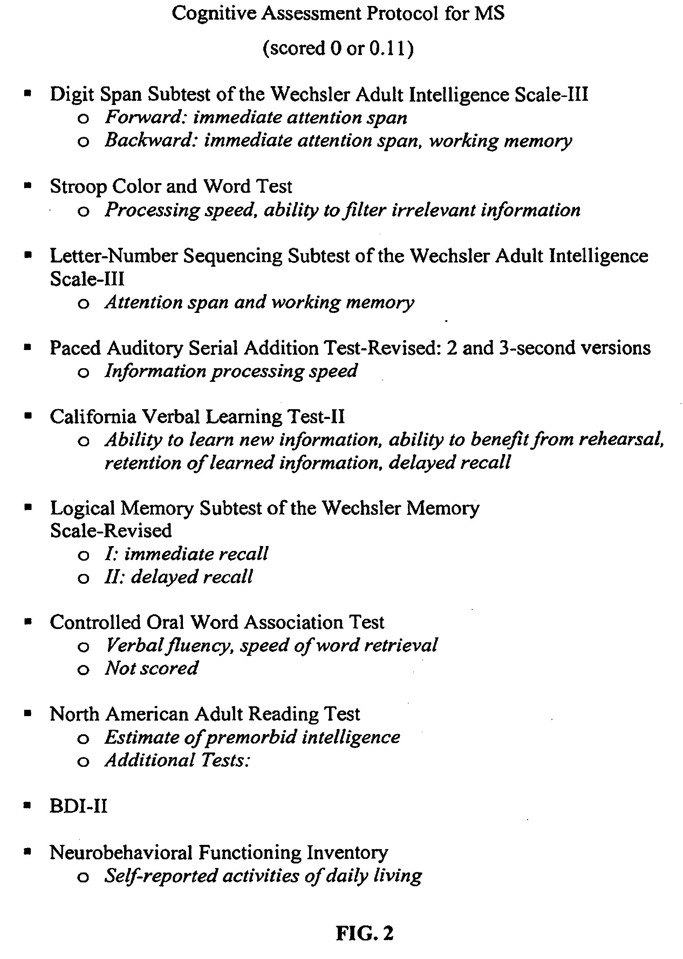

A longitudinal, multi-year, neuropsychological (NP) study has taken place on cognition in patients with MS who are tested specifically for the NP deficits typical of MS; generally testing occurs every one to two years. The NP battery consisted of eight (8) tests, which were scored as: Non-impaired = 0 or Impaired = 0.11 (> 1 SD from the norm); the eight tests that constituted the Cognitive Assessment Protocol for MS are set forth in Figure 2.

An individual was considered as cognitively impaired for the purpose of analysis if the total score of the eight (8) tests was ≥ 0.44 (Wilken JA, et al. Mult Scler. 2003;9:119-127). The Beck Depression Inventory-II (BDI-II) was used to exclude any individual with clinically significant depression.

A subset of the patients in the longitudinal study also began to use the drug Fampridine-SR. Accordingly, a retrospective chart review was conducted in patients in a Fampridine-SR open-label study who had NP testing done at least 6 months apart. The subset of patients reviewed had received Fampridine-SR for at least 3 months.

Thus, MS patients with cognitive impairment who participated in certain Fampridine-SR studies (e.g., MS-F203 and MS-F204), and who also had taken a comprehensive neuropsychological battery before beginning the blinded part of the study and who had taken a repeat neuropsychological battery at least 3 months after being enrolled in the open-label extension studies, were indentified. An impairment rating was assigned to each patient based on the results of each of eight neuropsychological tests specific for cognitive deficits typical in MS.

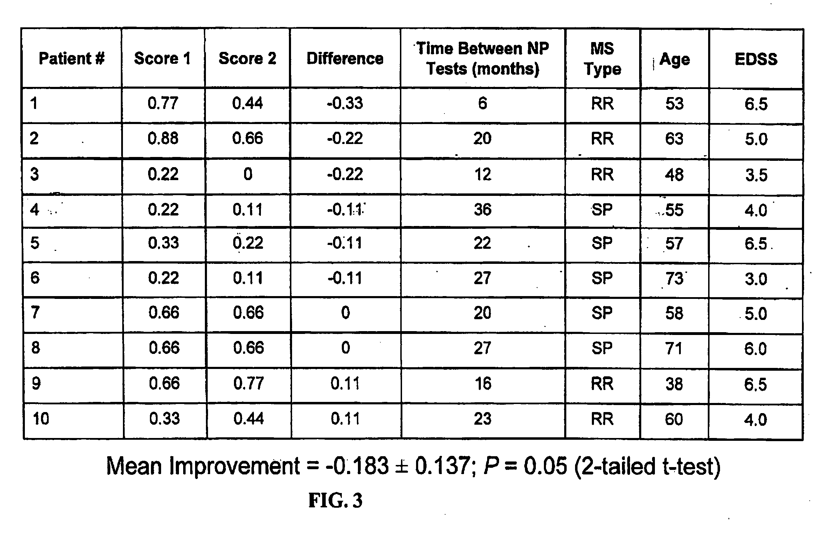

Results for the ten patients that had neuropsychological testing before entering either the MS-F203 or MS-F204 Fampridine-SR studies are set forth in Figure 3. These patients thereafter had repeated neuropsychological testing, after they had been on open-label Fampridine-SR 10 mg twice daily for more than three months. Of note, the 10 patients had a valid pre-study NP test and a follow-up NP test after three (3) months to one (1) year of treatment with Fampridine-SR without a change in disease modifying therapy or other significant concomitant medications. Of these 10 patients, six (6) cognitively improved, two (2) were unchanged, and two (2) declined; mean improvement = -0.183 ± 0.137; P = 0.05 (negative value means improvement).

Of these 10 patients, six (6) showed significant improvement in cognition, two (2) were unchanged and (2) showed mild decline, not inconsistent with the normal decline in cognitive function seen as MS progresses. Overall, the impairment ratings of the 10 patients showed improvement when on Fampridine-SR compared to their pre-treatment status (p=0.05). Fampridine-SR was useful as a symptomatic treatment in MS patients with cognitive impairment. Patients and their families have reported improved NP function, such as ability to carry on conversations, stay on a subject, and complete a thought or task. Retrospective analysis of a small number of patients showed improvement in NP testing.

### Example 2 - Kits:

Kits are also disclosed. The kit can comprise an outer receptacle or container configured to receive one or more inner receptacles/containers, utensils and/or instructions. One receptacle of the disclosure can be a bottle, blister pack, or box configured to contain, e.g., pills, capsules or tablets of the invention. A composition of the invention can be comprised within a receptacle of the disclosure. A receptacle of the disclosure can contain sufficient quantity of a composition of the invention to be useful for multiple doses, or may be in unit or single dose form. A utensil in accordance with the disclosure can comprise item(s) to administer the drug, such as a patch, inhalation apparatus, fluid container, cup, syringe or needle. Kits of the disclosure generally comprise instructions for administration in accordance with the present disclosure. Any mode of administration set forth or supported herein can constitute some portion of the instructions. The instructions indicate that the composition of the invention is to be taken twice daily. The instructions indicate that the composition of the invention is in 10 mg tablets of sustained release 4AP and that the patient is to take one 10 mg tablet twice daily. The instructions may be affixed to any container/receptacle of the disclosure. Alternatively, the instructions can be printed on or embossed in or formed as a component of a receptacle of the disclosure. A kit will also include instructions for employing the kit components as well the use of any other reagent not included in the kit. It is contemplated that such reagents are embodiments of kits of the disclosure. The instructions can include, e.g., information about taking the pills with liquid or food. Such kits, however, are not limited to the particular items identified above and may include any reagent used directly or indirectly in the treatment of cognitive dysfunction or cognitive impairment.

## Claims

1. A sustained release composition of 4-aminopyridine for use in a method of dosing to improve at least one neuro-cognitive or neuro-psychiatric parameter in a patient, wherein the patient has multiple sclerosis.

2. The composition for use according to claim 1, wherein the patient is human.

3. The composition for use according to claim 1 or 2, said composition to be administered twice daily in a dose of 10 milligrams of 4-aminopyridine.

4. The composition for use according to any one of claims 1-3, wherein the composition is in the form of a tablet, a pill or a capsule.

5. The composition for use according to claim 4, wherein the composition is in the form of a tablet.

6. The composition for use according to any one of claims 1-5, wherein the neuro-cognitive or neuro-psychiatric parameter is assayable by a neuropsychological test, wherein the neuropsychological test is Digit Span Subtest of the Wechsler Adult Intelligence Scale-III, Stroop Color and Word Test, Letter-Number Sequencing Subtest of the Wechsler Adult Intelligence Scale-III, Paced Auditory Serial Addition Test-Revised; California Verbal Learning Test-II, Logical Memory Subtest of the Wechsler Memory, Controlled Oral Word Association Test, or North American Adult Reading Test.

7. The composition for use according to claim 6, wherein the neuro-cognitive or neuro-psychiatric parameter is an impairment in attention span, an impairment in working memory, an impairment in memory wherein the impairment in memory is an impairment in immediate recall and/or delayed recall, an impairment in information processing speed and/or ability to filter irrelevant information, an impairment in verbal fluency and/or speed of word retrieval, or an impairment in ability to learn new information and/or ability to retain learned information.

8. The composition for use according to any one of claims 1-5, wherein the neuro-cognitive or neuro-psychiatric parameter is depression, altered libido or euphoria.

9. The composition for use according to any one of claims 1-8, wherein the patient has been diagnosed with an impairment or alteration in brain function including a disease-related neuro-psychiatric condition.

10. The composition for use according to any one of claims 1-9, wherein the composition is for administration in combination with a second therapy for treating the neuro-cognitive or neuro-psychiatric parameter, optionally, the second therapy is an anti-psychotic compound, a cholinesterase inhibitor, a nicotinic receptor agonist, or a nicotinic receptor antagonist.

11. Use of 4-aminopyridine in the manufacture of a sustained release medicament for dosing to improve at least one neuro-cognitive or neuro-psychiatric parameter in a patient, wherein the patient has multiple sclerosis.

12. The use of claim 11 further comprising the feature(s) of any one of claims 1-10.

## Patentansprüche

1. Zusammensetzung von 4-Aminopyridin mit anhaltender Freisetzung zur Verwendung bei einem Verabreichungsverfahren zur Verbesserung mindestens eines neurokognitiven oder neuropsychiatrischen Parameters bei einem Patienten, wobei der Patient multiple Sklerose hat.

2. Zusammensetzung zur Verwendung nach Anspruch 1, wobei der Patient ein Mensch ist.

3. Zusammensetzung zur Verwendung nach Anspruch 1 oder 2, wobei die Zusammensetzung zweimal täglich in einer Dosis von 10 mg 4-Aminopyridin verabreicht wird.

4. Zusammensetzung zur Verwendung nach einem der Ansprüche 1-3, wobei die Zusammensetzung in Form einer Tablette, einer Pille oder einer Kapsel vorliegt.

5. Zusammensetzung zur Verwendung nach Anspruch 4, wobei die Zusammensetzung in Form einer Tablette vorliegt.

6. Zusammensetzung zur Verwendung nach einem der Ansprüche 1-5, wobei der neurokognitive oder neuropsychiatrische Parameter durch einen neuropsychologischen Test untersucht werden kann, wobei es sich bei dem neuropsychologischen Test um den Digit Span Subtest des Wechsler Adult Intelligence Scale-III, den Stroop Color and Word Test, den Letter-Number Sequencing Subtest des Wechsler Adult Intelligence Scale-III, den Paced Auditory Serial Addition Test-Revised, den California Verbal Learning Test-II, den Logical Memory Subtest des Wechsler Memory, den Controlled Oral Word Association Test oder den North American Adult Reading Test handelt.

7. Zusammensetzung zur Verwendung nach Anspruch 6, wobei es sich bei dem neurokognitiven oder neuropsychiatrischen Parameter um eine Störung bei der Aufmerksamkeitsspanne, eine Störung des Arbeitsgedächtnis, eine Gedächtnisstörung, wobei es sich bei der Gedächtnisstörung um eine Störung der unmittelbaren Erinnerung und/oder der verzögerten Erinnerung handelt, eine Störung bei der Informationsverarbeitungsgeschwindigkeit und/oder der Fähigkeit zum Ausfiltern irrelevanter Informationen, eine Störung des Redeflusses und/oder der Geschwindigkeit des Abrufs von Wörtern oder eine Störung bei der Fähigkeit zum Lernen neuer Informationen und/oder der Fähigkeit zum Behalten gelernter Informationen handelt.

8. Zusammensetzung zur Verwendung nach einem der Ansprüche 1-5, wobei es sich bei dem neurokognitiven oder neuropsychiatrischen Parameter um Depression, eine geänderte Libido oder Euphorie handelt.

9. Zusammensetzung zur Verwendung nach einem der Ansprüche 1-8, wobei bei dem Patienten eine Störung oder Veränderung der Gehirnfunktion einschließlich eines mit einer Krankheit in Zusammenhang stehenden neuropsychiatrischen Leidens diagnostiziert wurde.

10. Zusammensetzung zur Verwendung nach einem der Ansprüche 1-9, wobei die Zusammensetzung für die Verabreichung in Kombination mit einer zweiten Therapie zur Behandlung des neurokognitiven oder neuropsychiatrischen Parameters bestimmt ist, wobei es sich bei der zweiten Therapie gegebenenfalls um eine antipsychotische Verbindung, einen Cholinesterasehemmer, einen Agonisten am nikotinischen Rezeptor oder einen Antagonisten am nikotinischen Rezeptor handelt.

11. Verwendung von 4-Aminopyridin bei der Herstellung eines Medikaments mit anhaltender Freisetzung zur Verabreichung zur Verbesserung mindestens eines neurokognitiven oder neuropsychiatrischen Parameters bei einem Patienten, wobei der Patient multiple Sklerose hat.

12. Verwendung nach Anspruch 11, welche weiterhin das/die Merkmale(e) eines der Ansprüche 1-10 umfasst.

## Revendications

1. Composition à libération prolongée de 4-aminopyridine pour utilisation dans un procédé d'administration pour améliorer au moins un paramètre neurocognitif ou neuropsychiatrique chez un patient, le patient ayant la sclérose en plaques.

2. Composition pour utilisation selon la revendication 1, le patient étant humain.

3. Composition pour utilisation selon la revendication 1 ou 2, ladite composition étant destinée à être administrée deux fois par jour à une dose de 10 milligrammes de 4-aminopyridine.

4. Composition pour utilisation selon l'une quelconque des revendications 1 à 3, la composition étant sous la forme d'un comprimé, d'une pilule ou d'une capsule.

5. Composition pour utilisation selon la revendication 4, la composition étant sous la forme d'un comprimé.

6. Composition pour utilisation selon l'une quelconque des revendications 1 à 5, le paramètre neurocognitif ou neuropsychiatrique pouvant être évalué par un test neuropsychologique, le test neuropsychologique étant le test de mémoire de chiffres de l'échelle d'intelligence de Wechsler III, le test de couleur et de mot de Stroop, le test de séquence de lettres-chiffres de l'échelle d'intelligence de Wechsler III, le test PASAT (Paced Auditory Serial Addition Test) révisé ; le test de mémoire verbale California II, le test de mémoire logique de l'échelle de mémoire de Wechsler, le test oral d'association de mots contrôlé, ou le test de lecture de l'adulte nord-américain.

7. Composition pour utilisation selon la revendication 6, le paramètre neurocognitif ou neuropsychiatrique étant une diminution de la durée d'attention, une diminution de la mémoire de travail, une diminution de la mémoire, la diminution de la mémoire étant une diminution du rappel immédiat et/ou du rappel différé, une diminution de la vitesse et/ou capacité de traitement d'information pour filtrer les informations non pertinentes, une diminution de la fluidité et/ou vitesse verbale de recherche de mots ou une diminution de la capacité d'apprentissage de nouvelles informations et/ou de la capacité à mémoriser des informations apprises.

8. Composition pour utilisation selon l'une quelconque des revendications 1 à 5, le paramètre neurocognitif ou neuropsychiatrique étant une dépression, une libido altérée ou une euphorie.

9. Composition pour utilisation selon l'une quelconque des revendications 1 à 8, le patient ayant été diagnostiqué avec une diminution ou altération de la fonction cérébrale comprenant un état neuropsychiatrique pathologique.

10. Composition pour utilisation selon l'une quelconque des revendications 1 à 9, la composition étant pour administration en combinaison avec une deuxième thérapie pour traiter le paramètre neurocognitif ou neuropsychiatrique, facultativement, la deuxième thérapie étant un composé antipsychotique, un inhibiteur de cholinestérase, un agoniste de récepteur nicotinique, ou un antagoniste de récepteur nicotinique.

11. Utilisation de 4-aminopyridine dans la fabrication d'un médicament à libération prolongée pour administration pour améliorer au moins un paramètre neurocognitif ou neuropsychiatrique chez un patient, le patient ayant la sclérose en plaques.

12. Utilisation de la revendication 11 comprenant en outre la/les caractéristique(s) de l'une quelconque des revendications 1 à 10.
